# EUROPEAN PATENT APPLICATION

(11) **EP 4 353 229 A1**
(43) Date of publication of application: **17.04.2024**
(21) Application number: 22806872.2
(22) Date of filing: 16.05.2022
(51) Int. Cl.: A61K 31/44, A61P 1/16, A61P 31/20

(54) **APPLICATION OF HYDRONIDONE IN PREPARATION OF DRUG FOR TREATING OR PREVENTING CHRONIC HEPATITIS B WITH LIVER FIBROSIS**

(30) Priority: 14.05.2021 CN 202110531848
(71) Applicant: Beijing Continent Pharmaceuticals Co., Ltd., Beijing 101300 (CN)
(72) Inventor: LUO, Ying, Shanghai 201203 (CN); MA, Songjiang, Beijing 100102 (CN)
(74) Representative: Gille Hrabal Partnerschaftsgesellschaft mbB Patentanwälte
(86) International application number: PCT/CN2022/093039
(87) International publication number: WO 2022/237910

(57) **Abstract**

The present invention provides a use of a compound represented by formula (I) and a solvate, hydrate, prodrug or pharmaceutically acceptable salt thereof in the preparation of a drug for treating or preventing chronic hepatitis B with liver fibrosis. Clinical research proves that the compound may effectively reverse liver fibrosis, and in particular has a better effect on treating fibrosis in a portal region, significant fibrosis, liver fibrosis in a progressive stage or liver cirrhosis.

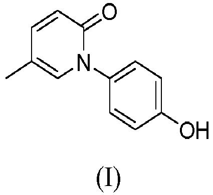

## Description

The present disclosure claims priority to Chinese Patent Application No. 202110531848.7 filed with China National Intellectual Property Administration on May 14, 2021 and entitled "APPLICATION OF HYDRONIDONE IN PREPARATION OF DRUG FOR TREATING OR PREVENTING CHRONIC HEPATITIS B WITH LIVER FIBROSIS", which is incorporated herein by reference in its entirety.

### TECHNICAL FIELD

The present disclosure belongs to the field of medicines, and particularly relates to use of hydronidone for manufacturing a medicament for the treatment or prevention of chronic hepatitis B with hepatic fibrosis.

### BACKGROUND

Hepatic fibrosis is a common pathological basis in the progress of chronic liver diseases. Various chronic injuries cause degeneration and necrosis of liver cells, and the fibrous connective tissue abnormally proliferates, excessively deposits, and wraps regenerated liver cells, forming a "pseudolobule" to destroy the original tissue structure of the liver, finally enabling the liver to form a nodular shape and become hard. The function of the liver is then damaged or even completely disappears to form cirrhosis. Hepatic fibrosis is histologically reversible. Cirrhosis is difficult to reverse, but a small portion can still be reversed.

Hepatic fibrosis may be caused by various chronic diseases, such as chronic viral hepatitis, chronic alcoholism, cholestasis, metabolic disorder diseases caused by congenital enzyme defects, long-term exposure to poisons and medicines, and the like. Hepatic fibrosis and cirrhosis are one of the main reasons affecting the quality of life and medical expenses of patients with liver diseases. The market demand for liver-protecting drugs is increasing year by year.

China has the largest population of people with hepatitis B in the world, which is about 70 million, wherein the patients with chronic hepatitis B are about 20 million-30 million, the cases of hepatitis B-related cirrhosis are about 1 million, and the cases of hepatitis B-related liver cancer are about 300 thousand. In 2016, a survey showed that the mortality of hepatitis B-related cirrhosis was 5.82/100 thousand people, and the mortality of hepatitis B-related liver cancer reached 16.42/100 thousand people. Hepatitis B seriously harms the health of people in China. Hepatic fibrosis is a key pathological process for the development of chronic hepatitis B to cirrhosis. Reversing or delaying the natural process of hepatic fibrosis can significantly reduce the occurrence of cirrhosis and liver cancer, reduce the death of patients, and reduce the heavy economic burden of the country and the society.

At present, no specific and effective anti-hepatic fibrosis therapeutic drugs exist in clinic. Hepatic injury and inflammation are relieved and hepatic fibrosis is prevented and treated mainly by treating basic diseases causing hepatic injury. The hepatic fibrosis treatment includes hepatic fibrosis etiology treatment and anti-hepatic fibrosis treatment. For chronic viral hepatitis B hepatic fibrosis, the continuous damage of the liver can be relieved by inhibiting and eliminating HBV, and the repair of fibrotic liver tissues is promoted to a certain extent. However, for hepatitis B hepatic fibrosis/cirrhosis patients, only antiviral therapy cannot completely solve the problem of hepatic fibrosis. The annual incidence rate of the compensated cirrhosis progressing to decompensated cirrhosis is 3%-5%, the 5-year survival rate of the decompensated cirrhosis is only 14%-35%, and the annual incidence rate of hepatocellular carcinoma of the cirrhosis patients is 3%-6%. Therefore, there is an urgent need for effective anti-hepatic fibrosis drugs. Particularly, there are few reports on drugs for treating or preventing chronic viral hepatitis B progressive hepatic fibrosis or compensated cirrhosis, and patients and doctors are urgently required to find safe and effective therapeutic drugs.

### SUMMARY

The present disclosure provides use of a compound of formula (I) (that is, hydronidone), a solvate, a hydrate, a prodrug, or a pharmaceutically acceptable salt thereof for manufacturing a medicament for the treatment and/or prevention of chronic hepatitis B with hepatic fibrosis,

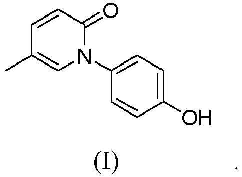

The present disclosure further provides a compound of formula (I), a solvate, a hydrate, a prodrug, or a pharmaceutically acceptable salt thereof for use in the treatment and/or prevention of chronic hepatitis B with hepatic fibrosis.

The present disclosure further provides a method of treatment and/or prevention of chronic hepatitis B with hepatic fibrosis, comprising the step of administering to a patient in need thereof a therapeutically or prophylactically effective amount of the compound of formula (I), the solvate, the hydrate, the prodrug, or the pharmaceutically acceptable salt thereof.

According to some embodiments of the present disclosure, the chronic hepatitis B with hepatic fibrosis may be portal area fibrosis, significant hepatic fibrosis, progressive hepatic fibrosis, or cirrhosis. According to some embodiments of the present disclosure, the cirrhosis may be compensated cirrhosis or decompensated cirrhosis.

According to some embodiments of the present disclosure, the portal area fibrosis may be characterized via an Ishak scoring system, which may mean, for example, hepatic fibrosis with an Ishak score of ≥ 1. For example, hepatic fibrosis with an Ishak score of ≥ 1, an Ishak score of ≥ 2, or an Ishak score of about 1-3, about 1-2, or about 2-3, or the like.

According to some embodiments of the present disclosure, the portal area fibrosis may be characterized via a Scheuer scoring system, which may mean, for example, hepatic fibrosis with a Scheuer score of about ≥ 1, or hepatic fibrosis with a Scheuer score of about 1-2.

According to some embodiments of the present disclosure, the portal area fibrosis may be characterized via a METAVIR scoring system, which may mean, for example, hepatic fibrosis with a METAVIR score of about ≥ 1, or hepatic fibrosis with a METAVIR score of about 1-2.

According to some embodiments of the present disclosure, the portal area fibrosis may be characterized via a Knodell scoring system, which may mean, for example, hepatic fibrosis with a Knodell score of about ≥ 1, or hepatic fibrosis with a Knodell score of about 1-2.

According to some embodiments of the present disclosure, the significant hepatic fibrosis may be characterized via an Ishak scoring system, which may mean, for example, hepatic fibrosis with an Ishak score of ≥ 3. For example, hepatic fibrosis with an Ishak score of ≥ 4, or an Ishak score of about 3-6, about 3-5, about 3-4, about 4-6, about 4-5, or about 5-6, or the like.

According to some embodiments of the present disclosure, the significant hepatic fibrosis may be characterized via a Scheuer scoring system, which may mean, for example, hepatic fibrosis with a Scheuer score of about ≥ 2 or ≥ 3, or hepatic fibrosis with a Scheuer score of about 2-4, 3-4, or 2-3. According to some embodiments of the present disclosure, the significant hepatic fibrosis may be characterized via a METAVIR scoring system, which may mean, for example, hepatic fibrosis with a METAVIR score of about ≥ 2 or ≥ 3, or hepatic fibrosis with a METAVIR score of about 2-4, 3-4, or 2-3.

According to some embodiments of the present disclosure, the significant hepatic fibrosis may be characterized via a Knodell scoring system, which may mean, for example, hepatic fibrosis with a Knodell score of about ≥ 2 or ≥ 3, or hepatic fibrosis with a Knodell score of about 2-4, 3-4, or 2-3. According to some embodiments of the present disclosure, the significant hepatic fibrosis may be characterized by a liver stiffness measurement (LSM), which may mean, for example, hepatic fibrosis with an LSM of about ≥ 5.8 kPa, for example, hepatic fibrosis with an LSM of about 5.8-12.4 kPa, about 5.8-9.4 kPa, about 5.8-8 kPa, about 5.8-7.4 kPa, about 5.8-6 kPa, or about 9.4-12.4 kPa. The liver stiffness measurement may be measured using devices conventional in the art, such as a FibroTouch or FibroScan device.

According to some embodiments of the present disclosure, the progressive hepatic fibrosis may be characterized via an Ishak scoring system, which may mean, for example, hepatic fibrosis with an Ishak score of ≥ 4. For example, hepatic fibrosis with an Ishak score of about ≥ 5, such as about 4-6, about 4-5, or about 5-6.

According to some embodiments of the present disclosure, the progressive hepatic fibrosis may be characterized via a Scheuer scoring system, which may mean, for example, hepatic fibrosis with a Scheuer score of about ≥ 3 or about 3-4.

According to some embodiments of the present disclosure, the progressive hepatic fibrosis may be characterized via a METAVIR scoring system, which may mean, for example, hepatic fibrosis with a METAVIR score of about ≥ 3 or about 3-4.

According to some embodiments of the present disclosure, the progressive hepatic fibrosis may be characterized via a Knodell scoring system, which may mean, for example, hepatic fibrosis with a Knodell score of about ≥ 3 or about 3-4.

According to some embodiments of the present disclosure, the progressive hepatic fibrosis may be characterized by a liver stiffness measurement (LSM), for example, LSM of about ≥ 6.0 kPa, about ≥ 7.4 kPa, about ≥ 8.0 kPa, about ≥ 9.0 kPa, about ≥ 9.5 kPa, about ≥ 10.0 kPa, about ≥ 12.0 kPa, or about ≥ 12.4 kPa, for example, LSM of about 7.4-17.0 kPa, about 7.4-15.0 kPa, about 7.4-14.6 kPa, about 8.0-17.0 kPa, about 8.0-15.0 kPa, about 8.0-14.6 kPa, about 9.0-17.0 kPa, about 9.0-15.0 kPa, about 9.0-14.6 kPa, about 9.5-17.0 kPa, about 9.5-15.0 kPa, about 9.5-14.6 kPa, about 10.0-17.0 kPa, about 10.0-15.0 kPa, or about 10.0-14.6 kPa.

According to some embodiments of the present disclosure, the progressive hepatic fibrosis may be characterized by a liver stiffness measurement (LSM), for example, hepatic fibrosis with an LSM of about ≥ 9.0 kPa or ≥ 10.0 kPa under the condition that bilirubin is normal and ALT (alanine aminotransferase) is < 5 ULN, or cirrhosis with an LSM of about ≥ 6.0 kPa under the condition that bilirubin is normal and ALT is normal, such as hepatic fibrosis with an LSM of about ≥ 7.0 kPa, about ≥ 8.0 kPa, or about ≥ 9.0 kPa.

According to some embodiments of the present disclosure, the cirrhosis may be characterized via an Ishak scoring system, which may mean, for example, cirrhosis with an Ishak score of ≥ 5, or cirrhosis with an Ishak score of about 5-6.

According to some embodiments of the present disclosure, the cirrhosis may be characterized via a Scheuer scoring system, which may mean, for example, cirrhosis with a Scheuer score of about ≥ 4. According to some embodiments of the present disclosure, the cirrhosis may be characterized via a METAVIR scoring system, which may mean, for example, cirrhosis with a METAVIR score of about ≥ 4.

According to some embodiments of the present disclosure, the cirrhosis may be characterized via a Knodell scoring system, which may mean, for example, cirrhosis with a Knodell score of about ≥ 4. According to some embodiments of the present disclosure, the cirrhosis may be characterized by a liver stiffness measurement (LSM), for example, cirrhosis with an LSM of about ≥ 12.0 kPa, about ≥ 13.0 kPa, about ≥ 15.0 kPa, about ≥ 16.0 kPa, or about ≥ 17.0 kPa.

According to some embodiments of the present disclosure, the cirrhosis may also be classified into compensated cirrhosis and decompensated cirrhosis by one of the following three criteria: clinical staging based on major complications, staging based on hepatic venous pressure gradient (HVPG), and grading and staging of liver function reserve (Child-Pugh score grading and staging).

According to some embodiments of the present disclosure, the clinical staging based on major complications may further include a two-stage staging method, a four-stage staging method, a five-stage staging method, and a seven-stage staging method.

The criteria of the two-stage staging method are that according to the natural history of cirrhosis, when severe complications such as ascites, gastroesophageal variceal bleeding (GEVB), hepatic encephalopathy (HE), and the like have not yet appeared, the cirrhosis is compensated cirrhosis, and when one of the complications described above appears, the cirrhosis is decompensated cirrhosis. The criteria of the four-stage staging method are that stage 1-2 is compensated cirrhosis, and stage 3-4 is decompensated cirrhosis, wherein, stage 1 is without gastroesophageal varices and ascites, stage 2 is with gastroesophageal varices and without ascites, stage 3 is with ascites with or without gastroesophageal varices, and stage 4 is with esophageal variceal bleeding (EVB) with or without ascites.

The criteria of the five-stage staging method are that stage 1-2 is compensated cirrhosis, and stage 3-5 is decompensated cirrhosis.

The criteria of the seven-stage staging method are that stage 0-2 is compensated cirrhosis, and stage 3-6 is decompensated cirrhosis, wherein, stage 0 is without gastroesophageal varices, and HVPG is between 5-10 mmHg; stage 1 is without gastroesophageal varices, but HVPG is ≥ 10 mmHg; stage 2 is with esophagogastric fundal varices; stage 3 is with EVB; in stage 4, a decompensation event other than bleeding occurs for the first time; in stage 5, a decompensation event occurs again; and in stage 6 that is a late decompensated stage, refractory ascites, HE, severe infection, renal failure, and chronic plus acute liver failure occur.

According to some embodiments of the present disclosure, when HVPG is ≤ 12 mmHg, the cirrhosis is compensated cirrhosis, and when HVPG is > 12 mmHg, the cirrhosis is decompensated cirrhosis, wherein when HVPG is < 10 mmHg, varices are absent, and when HVPG is between 10-12 mmHg, clinically significant portal hypertension is present.

According to some embodiments of the present disclosure, the Child-Pugh grading is that grade A is compensated cirrhosis, and grade B or grade C in the Child-Pugh grading is decompensated cirrhosis, wherein grade B is an early decompensated stage, and grade C is a late decompensated stage. According to some embodiments of the present disclosure, the chronic hepatitis B with hepatic fibrosis has not progressed to liver cancer.

The Ishak scoring system is an improved version of the Knodell scoring system, which divides hepatic fibrosis evaluation into stages 0-6, and is the most sensitive and most common method internationally at present for evaluating hepatic fibrosis for a before-after case-control study. According to the consensus on diagnosis and treatment of hepatic fibrosis (2019) published by the Hepatology Branch of the Chinese Medical Association, the Gastroenterology Branch of the Chinese Medical Association, and the Infectious Diseases Branch of the Chinese Medical Association in combination (Journal of Practical Hepatology, November 2019, 22(6):793-803), in recent years, scholars at home and abroad have quantified the terms "minority" and "majority" in the Ishak score to reduce human error. They have staged it based on fibrous intervals and the number of pseudolobules. "Minority" refers to 1-3 fibrous intervals, and more than 3 is considered "majority". Therefore, the Ishak scoring system expresses staging of hepatic fibrosis (i.e., Ishak score) as follows: stage I (Ishak score 1): no fibrous interval; stage II (Ishak score 2): 1 fibrous interval; stage III (Ishak score 3): 2-3 fibrous intervals; stage IV (Ishak score 4): 4 fibrous intervals; stage V (Ishak score 5): not less than 4 fibrous intervals + 1-3 explicit pseudolobules; and stage VI (Ishak score 6): more than 3 pseudolobules.

According to some embodiments of the present disclosure, an Ishak score of 0 can indicate no fibrosis. In some embodiments, an Ishak score of 1 can indicate that some portal areas are fibrotic (PF), with or without a short fibrous interval. In some embodiments, an Ishak score of 2 can indicate that most of portal areas are fibrotic, with or without 1 fibrous interval. In some embodiments, an Ishak score of 3 can indicate that most of portal areas are fibrotic, with 2-3 fibrous intervals. In some embodiments, an Ishak score of 4 can indicate portal area fibrosis with significant portal-to-portal bridging fibrosis and portal-to-central bridging fibrosis, with not less than 4 fibrous intervals. In some embodiments, an Ishak score of 5 can indicate a significant portal-to-portal bridging fibrosis and/or portal-to-central bridging fibrosis, with 1-3 pseudolobules. In some embodiments, an Ishak score of 6 can indicate more than 3 pseudolobules, indicating cirrhosis.

According to some embodiments of the present disclosure, the Ishak score may be converted to another scoring system. The scoring system may be, for example, a Scheuer score, a METAVIR score, a Knodell score, or the like.

The Scheuer scoring system divides hepatic fibrosis into stages 0-4. According to some embodiments of the present disclosure, a Scheuer score of 0 may indicate no fibrosis. In some embodiments, a score of 1 may indicate enlargement of the portal area. In some embodiments, a score of 2 may indicate portal area fibrosis (PF), with formation of a fibrous interval. In some embodiments, a score of 3 may indicate a fibrous interval with a disordered lobule structure. In some embodiments, a score of 4 may indicate cirrhosis. In some embodiments, the Scheuer score may be ≥ 1, ≥ 2, or ≥ 3, or may be 0-4, 1-4, 2-4, 3-4, 1-3, or 2-3.

The METAVIR scoring system divides hepatic fibrosis into stages 0-4. According to some embodiments of the present disclosure, a METAVIR score of F0 may indicate no fibrosis. In some embodiments, a METAVIR score of 1 may indicate portal area fibrosis without a fibrous interval. In some embodiments, a METAVIR score of 2 may indicate portal area fibrosis with a few fibrous intervals. In some embodiments, a METAVIR score of 3 may indicate an interval fibrosis and a disordered structure. In some embodiments, a METAVIR score of 4 may indicate cirrhosis. In some embodiments, the METAVIR score may be ≥ 1, ≥ 2, or ≥ 3, or may be 0-4, 1-4, 2-4, 3-4, 1-3, 1-2, or 2-3.

The Knodell scoring system divides hepatic fibrosis into stages 0-4. According to some embodiments of the present disclosure, a Knodell score of 0 may indicate no fibrosis. In some embodiments, a Knodell score of 1 may indicate mild fibrosis and enlargement of the portal area. In some embodiments, a Knodell score of 2 may indicate moderate fibrosis. In some embodiments, a Knodell score of 3 may indicate severe fibrosis, portal-to-portal bridging fibrosis, and portal-to-central bridging fibrosis. In some embodiments, a Knodell score of 4 may indicate cirrhosis. In some embodiments, the Knodell score may be ≥ 1, ≥ 2, or ≥ 3, or may be 1-4, 1-3, 2-4, or 2-3.

The Child-Pugh grading criteria are grading criteria commonly used clinically for quantitative evaluation of liver reserve function of a cirrhosis patient, different states of 5 indexes (including general conditions, ascites, serum bilirubin, serum albumin concentration, and prothrombin time, and the general conditions can be replaced by the existence and degree of hepatic encephalopathy) of the patient are divided into three levels, 1 point, 2 points, and 3 points are respectively recorded, and the scores of the 5 indexes are added. The sum is 5 points at the lowest and 15 points at the highest. The liver reserve function is divided into three grades, A, B, and C, according to the sum, wherein grade A is 5-6 points, grade B is 7-9 points, and grade C is 10-15 points. The higher the score, the worse the liver reserve function.

According to an embodiment of the present disclosure, the compound of formula (I) may be used in an amount of 50-500 mg/day, such as 80-450 mg/day, 100-400 mg/day, or 120-360 mg/day, illustratively 180 mg/day, 270 mg/day, or 360 mg/day.

According to an embodiment of the present disclosure, the medicament may be administered 1-5 times daily, for example, 3 times daily.

According to an embodiment of the present disclosure, the pharmaceutically acceptable salt includes salts formed by an ester formed by the compound of formula (I) with an organic acid selected from propionic acid, oxalic acid, malonic acid, succinic acid, fumaric acid, maleic acid, lactic acid, malic acid, tartaric acid, and citric acid, or an acidic amino acid selected from aspartic acid and glutamic acid, with an inorganic base, including sodium, potassium, calcium, aluminium and ammonium salts, or salts formed with an organic base, including methylamine, ethylamine and ethanolamine salts; or salts formed by an ester formed by the compound of formula (I) with a basic amino acid selected from lysine, arginine, and ornithine, with an inorganic acid selected from hydrochloric acid, hydrobromic acid, hydrofluoric acid, sulfuric acid, nitric acid, and phosphoric acid, or salts formed with an organic acid selected from formic acid, acetic acid, picric acid, methanesulfonic acid, and ethanesulfonic acid. According to an embodiment of the present disclosure, the medicament further comprises one or more pharmaceutically acceptable carriers or excipients.

According to an embodiment of the present disclosure, the medicament may be an oral preparation or a parenteral preparation. Further, the oral preparation may be tablets, capsules, pills, dispersible powders, granules, oral liquids, syrups, elixirs, or the like; the parenteral preparation may be an injection, an powder injection, or the like.

Certain examples of suitable excipients include lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup, and methylcellulose. The preparation may further comprise a lubricant such as talc, magnesium stearate, and mineral oil; a wetting agent; an emulsifier and a suspending agent; a preservative such as methyl benzoate and hydroxypropyl benzoate; a sweetening agent and a flavoring agent. The medicament of the present disclosure may be formulated by the methods known in the art so as to provide immediate, sustained, or delayed release of the active ingredient after administration to the patient.

The tablets or pills of the present disclosure may be coated or compounded to obtain a dosage form affording the advantage of long-acting effect. For example, the tablets or pills contain an inner dosage component and an outer dosage component, the latter being in the form of an envelope of the former. The two components may be separated by an enteric-coated layer which serves to prevent the disintegration in the stomach to allow the inner component to pass through the duodenum entirely or to be delayed in release. A variety of substances may be used for such enteric-coated layers or coatings, including various polymeric acids and mixtures of polymeric acids and such substances as shellac, cetyl alcohol and cellulose acetate. Liquid forms in which the compound of the present disclosure may be incorporated for administration orally or by injection include aqueous solutions, suitably flavoured syrups, aqueous or oil suspensions; and emulsions formulated with edible oils such as cottonseed oil, sesame oil, medium-chain oil, coconut oil, or peanut oil; and elixirs and similar pharmaceutical vehicles.

A pharmaceutical composition comprises the compound of formula (I) or the pharmaceutically acceptable salt thereof, lactose monohydrate, silica, and magnesium stearate.

According to an embodiment of the present disclosure, in weight percentage, the compound of formula (I) or the pharmaceutically acceptable salt thereof has a content of 20-30%, the lactose monohydrate has a content of 70-80%, the silica has a content of 0.1-1%, and the magnesium stearate has a content of 0.1-1%.

### Beneficial Effects

The present disclosure provides use of a compound of formula (I), a solvate, a hydrate, a prodrug, or a pharmaceutically acceptable salt thereof for manufacturing a medicament for the treatment or prevention of chronic hepatitis B with hepatic fibrosis, which can effectively reverse hepatic fibrosis, and particularly has a better effect in the treatment of significant fibrosis, progressive hepatic fibrosis, or cirrhosis.

### BRIEF DESCRIPTION OF THE DRAWING

FIG. 1 shows an Ishak score distribution of hepatic fibrosis before and after treatment (FAS).
FIG. 2 shows an Ishak score distribution of hepatic fibrosis before and after treatment (PPS).
FIG. 3 shows the difference of the hepatic fibrosis Ishak score after 52 weeks of treatment decreased by >_ 1 point compared with the baseline between the groups (FAS).
FIG. 4 shows the difference of the hepatic fibrosis Ishak score after 52 weeks of treatment decreased by >_ 1 point compared with the baseline between the groups (PPS).

### DETAILED DESCRIPTION

The technical solutions of the present disclosure will be further described in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products, or can be prepared by using known methods.

### Preparation Example: Preparation of Hydronidone Capsules

**Formula:** Formula of the unit dose product is detailed in the following table:

| **Component** | **Content** | **Effect** |
|---|---|---|
| Hydronidone | 30mg | Active ingredient |
| Lactose monohydrate | 90mg | Filler |
| Silica | 0.6mg | Glidant |
| Magnesium stearate | 0.6mg | Lubricant |
| No. 4 empty gelatin capsule | 1 capsule | Capsule shell |

### Preparation method:

The detailed production process is described below, with a process verification batch (batch: 900 thousand capsules) as a representative:
**1) Preparing raw and auxiliary materials:**
   - Getting the raw and auxiliary materials, carefully checking the name, lot number, weight, manufacturer, etc., and checking that the package is intact.
   - Sieving lactose monohydrate, magnesium stearate, silica, and hydronidone respectively through a 80-mesh sieve for later use.
**2) Weighing and proportioning:** Accurately weighing each raw and auxiliary material according to the batch instructions and rechecking.
**3) Mixing:** Adding all the materials into a three-dimensional motion mixer, starting the mixer, slowly increasing the rotation speed to 800 r/min, mixing for 15 min to obtain a mixed powder intermediate product, and checking the material balance to be 98.00%-100.00%.
**4) Filling capsules:** Using a No. 4 capsule automatic hard capsule filler, adjusting the filling amount according to the content of the intermediate product, and filling with the weight difference within a specified range.
**5) Polishing and selecting capsules:** Polishing the filled capsules. Collecting the polished capsules, placing them in a tray, sieving to remove fine powder attached on the surface of the capsules, and picking out unqualified products with a plum blossom-shaped cap, a wrinkle, a notch, an air bubble, breakage, a shrunken cap, or double caps. Checking the material balance to be 98.00%-100.00%.
**6) Inner packaging:** Bottling and labeling, 63 capsules/bottle. Checking the material balance to be 98.00%-100.00%.
**7) Packaging.**

### Efficacy Example

### 1. Design of experiment

### 1.1. Overall design of experiment

This is a randomized, double-blind, placebo-controlled, multicenter, and dose-finding phase II clinical study with foundational treatment. In the clinical trial, three different dose test groups and a placebo control group were set on the basis of the entecavir capsule antiviral treatment, and the planned sample amount of each group was 60 cases, totaling 240 cases. The three dose test groups were 180 mg group, 270 mg group, and 360 mg group, respectively. The proportion of each test group to the control group was 1:1:1:1, and the cases were distributed randomly.

### 1.2. Subject selection

Inclusion criteria:
(1) Aged 18-65 years old, male or female
(2) A history of chronic hepatitis B, HBsAg positive ≥ 6 months
(3) ALT (alanine aminotransferase) < 8 times ULN (upper limit of normal)
(4) Liver biopsy confirmed significant hepatic fibrosis (Ishak score ≥ 3, except for decompensated cirrhosis)
(5) HBeAg positive patient, HBV DNA > 2.0×10⁴ IU/mL (10⁵ copies/mL);
   HBeAg negative patient, HBV DNA > 2.0×10³ IU/mL (10⁴ copies/mL)
(6) Not received interferon and/or nucleoside analogue antiviral treatment within 3 months before enrollment
(7) Not taken anti-inflammatory and liver-protecting drugs within 1 month before enrollment
(8) Not taken drugs with anti-fibrosis as main effect within 3 months before enrollment
(9) Able to understand and sign informed consent before study

Exclusion criteria:
(1) Not meeting any one of the inclusion criteria
(2) With upper gastrointestinal hemorrhage within 3 months before enrollment
(3) TBiL (total bilirubin) > 3 times ULN, or 3 ULN < ALT < 8 ULN and TBiL > 2 times ULN
(4) AFP (alpha-fetoprotein) >100 µg/L although no liver cancer indication
(5) PLT (platelet) ≤ 60 × 10⁹/L
(6) PTA (prothrombin activity) < 50%
(7) Obvious space-occupying lesion in liver shown by B-mode ultrasound, indicating a tumor
(8) The width of portal vein ≥ 1.2 cm shown by B-mode ultrasound
(9) Body mass index (BMI) > 30
(10) Patients with liver function decompensated cirrhosis and a liver tumor
(11) Patients with alcoholic, medicinal, genetic, immunological, and other viral and non-viral chronic hepatitis
(12) Patients combined with severe cardiovascular, pulmonary, renal, endocrine, nervous, and hematological diseases and patients with mental diseases
(13) Active peptic ulcer
(14) Women in pregnancy and lactation
(15) Participants in other drug trials within 3 months
(16) Patients considered by the investigator to be unsuitable for participation in the study Withdrawal criteria:
   (1) Subject self-withdrawal or withdrawal of informed consent
   (2) Subjects who were lost to follow-up
   (3) The investigator decided to terminate the treatment
   (4) The subject was pregnant or was determining to be pregnant
   (5) Any one of the following conditions occurred in the subject:
      ① ALT > 8 ULN;
      ② ALT > 5 ULN for 2 weeks;
      ③ ALT > 3 ULN and (TBiL > 3 ULN or INR (International normalized ratio) > 1.5 ULN);
      ④ ALT > 3 ULN with progressive fatigue, nausea, vomiting, pain or tenderness in the right upper abdomen, fever, rash, and/or eosinophilia (> 5%)
   (6) Decompensated cirrhosis occurred in the subject
   (7) Liver cancer occurred in the subject
   (8) Patients allergic to the medicine and patients experiencing serious adverse events, complications, or special physiological changes, who were not suitable to continue study participation, and the like

Dropout criteria:
(1) Enrollment by mistake
(2) Those who had not taken the medicine
(3) Without any test record
(4) Effectiveness and safety evaluation could not be performed due to the use of certain forbidden drugs
(5) Although study treatment was completed, the dosage was not in the range of 80%-120% of the dosage to be taken
(6) Discontinuation of the test drug for not less than cumulative 4 weeks
(7) Cases of accidental unblinding or emergent unblinding

### 1.3. Test drug

Both the hydronidone capsules and the placebo capsules were developed and provided by Shanghai Genomics, Inc., and they were identical in appearance. The specification of the hydronidone capsule was 30 mg/capsule. Test drug lot No.: 20150201 and 20170101.

Preservation of the test drug: room temperature. The validity period of the drug was 36 months.

All clinical test drugs were prepared by Beijing Continent Pharmaceuticals Co., Ltd in a GMP compliant workshop and were tested to be acceptable according to the quality standards approved by the National Medical Product Administration.

Entecavir capsules (0.5 mg/capsule, 7 capsules/strip/box, 24 months of validity period, lot No.: 1411102, 1506204, 1606201, 1606202, 1602205, 1612205, 1703202, 3031802, and 2031824), which were basic antiviral therapeutic drugs, were developed and manufactured by Fujian Cosunter Pharmaceutical Co., Ltd, and provided by Shanghai Genomics, Inc.

Preservation of the entecavir capsule: protected from light and sealed in a dry place at not more than 25 °C.

### Route of administration and dose setting:

According to animal pharmacodynamic experiments and phase I clinical trial results, the administration dose of the test drug of each group of the trial was determined as follows:
180 mg group: hydronidone capsule, 60 mg tid orally, for 52 consecutive weeks;
270 mg group: hydronidone capsule, 90 mg tid orally, for 52 consecutive weeks;
360 mg group: hydronidone capsule, 120 mg tid orally, for 52 consecutive weeks;
placebo group (control group): placebo capsule, 4 capsules each time, three times a day, orally, for 52 consecutive weeks;
Basic treatment: entecavir capsule, 0.5 mg each time, once a day, orally, for 52 consecutive weeks;
the placebo group and all the hydronidone groups were simultaneously subjected to entecavir basic treatment.

### 1.4. Procedures

### 1.4.1. Screening period/baseline

- Demographics: including date of birth, age, sex, ethnicity, height, weight, and occupation.
- The current history, the past history (cardiovascular, respiratory, digestive, urogenital, nervous, and mental systems, and the like), the history of drugs, and the addiction to tobacco, alcohol, and the like.
- The clinical symptoms were recorded.
- The informed consent was signed.
- The screening number was distributed.
- Physical examination.
- Routine laboratory examination: routine blood test, routine urinalysis, urine pregnancy (women of childbearing age), blood sugar, and the like.
- Serological examination: alanine aminotransferase (ALT), aspartate aminotransferase (AST), y-glutamyl transpeptidase (GGT), alkaline phosphatase (ALP), total protein (TP), albumin (A), globulin (G), total bilirubin (TBil), direct bilirubin (DBil), urea nitrogen (BUN), creatinine (Cr), triglyceride (TG), cholesterol (TC), low-density lipoprotein (LDL-C), high-density lipoprotein (HDL-C), prothrombin time (PT), prothrombin activity (PTA), and alpha-fetoprotein (AFP).
- Liver transient elastography LSM (kPa).
- Virology examination: HBsAg, anti-HBs, HBeAg, anti-HBe, anti-HBc, HBV DNA, and anti-HCV.
- Electrocardiogram: 12-lead electrocardiogram.
- Ultrasonic examination: liver, gallbladder, spleen, portal vein width, and/or blood flow rate, and the like.
- Percutaneous liver needle biopsy (results within 3 months before screening could be used as baseline data).
- The CRF was filled out.

### 1.4.2. Treatment period

### 1.4.2.1. Week 0

- The drug number was distributed.
- The test drug was dispensed.
- The concomitant medication was recorded.
- The adverse events were recorded.
- The CRF was filled out.

### 1.4.2.2. Week 1 (± 1 day)

- The dosing of the test drug was recorded.
- The concomitant medication was recorded.
- The adverse events were recorded.
- The CRF was filled out.

### 1.4.2.3. Week 4 (± 5 days)

- The test drug was recovered and dispensed.
- The dosing of the test drug was recorded.
- The concomitant medication was recorded.
- Physical examination.
- The clinical symptoms were recorded.
- Routine laboratory examination: routine blood test, routine urinalysis, and urine pregnancy (women of childbearing age).
- Serological examination: ALT, AST, GGT, ALP, TP, A, G, TBil, DBil, BUN, Cr, TG, TC, LDL-C, HDL-C, PT, PTA, and AFP.
- Virology examination: HBV DNA.
- The adverse events were recorded.
- The CRF was filled out.

### 1.4.2.4. Week 8 (± 5 days)

- The test drug was recovered and dispensed.
- The dosing of the test drug was recorded.
- The concomitant medication was recorded.
- Physical examination.
- The clinical symptoms were recorded.
- Serological examination: ALT, AST, GGT, ALP, TP, A, G, TBil, DBil, TG, TC, LDL-C, and HDL-C.
- Virology examination: HBV DNA.
- The adverse events were recorded.
- The CRF was filled out.

### 1.4.2.5. Week 12 (± 5 days)

- The test drug was recovered and dispensed.
- The dosing of the test drug was recorded.
- The concomitant medication was recorded.
- Physical examination.
- The clinical symptoms were recorded.
- Routine laboratory examination: routine blood test, routine urinalysis, urine pregnancy (women of childbearing age), blood sugar, and the like.
- Serological examination: ALT, AST, GGT, ALP, TP, A, G, TBil, DBil, BUN, Cr, TG, TC, LDL-C, HDL-C, PT, PTA, and AFP.
- Virology examination: HBV DNA.
- Electrocardiogram.
- The adverse events were recorded.
- The CRF was filled out.

### 1.4.2.6. Week 16 (± 5 days)

- The test drug was recovered and dispensed.
- The dosing of the test drug was recorded.
- The concomitant medication was recorded.
- Physical examination.
- The clinical symptoms were recorded.
- Serological examination: ALT.
- The adverse events were recorded.
- The CRF was filled out.

### 1.4.2.7. Week 20 (± 5 days)

- The test drug was recovered and dispensed.
- The dosing of the test drug was recorded.
- The concomitant medication was recorded.
- Physical examination.
- The clinical symptoms were recorded.
- Serological examination: ALT.
- The adverse events were recorded.
- The CRF was filled out.

### 1.4.2.8. Week 24 (± 5 days)

- The test drug was recovered and dispensed.
- The dosing of the test drug was recorded.
- The concomitant medication was recorded.
- Physical examination.
- The clinical symptoms were recorded.
- Routine laboratory examination: routine blood test, routine urinalysis, urine pregnancy (women of childbearing age), blood sugar, and the like.
- Serological examination: ALT, AST, GGT, ALP, TP, A, G, TBil, DBil, BUN, Cr, TG, TC, LDL-C, HDL-C, PT, PTA, and AFP.
- Liver transient elastography LSM (kPa).
- Virology examination: HBV DNA.
- Electrocardiogram.
- Ultrasonic examination: liver, gallbladder, spleen, portal vein width, and/or blood flow rate, and the like.
- The adverse events were recorded.
- The CRF was filled out.

### 1.4.2.9. Week 28 (± 5 days)

- The test drug was recovered and dispensed.
- The dosing of the test drug was recorded.
- The concomitant medication was recorded.
- Physical examination.
- The clinical symptoms were recorded.
- The adverse events were recorded.
- The CRF was filled out.

### 1.4.2.10. Week 32 (± 5 days)

- The test drug was recovered and dispensed.
- The dosing of the test drug was recorded.
- The concomitant medication was recorded.
- Physical examination.
- The clinical symptoms were recorded.
- The adverse events were recorded.
- The CRF was filled out.

### 1.4.2.11. Week 36 (± 5 days)

- The test drug was recovered and dispensed.
- The dosing of the test drug was recorded.
- The concomitant medication was recorded.
- Physical examination.
- The clinical symptoms were recorded.
- Routine laboratory examination: routine blood test, routine urinalysis, urine pregnancy (women of childbearing age), blood sugar, and the like.
- Serological examination: ALT, AST, GGT, ALP, TP, A, G, TBil, DBil, BUN, Cr, TG, TC, LDL-C, HDL-C, PT, PTA, and AFP.
- Virology examination: HBV DNA.
- Electrocardiogram.
- The adverse events were recorded.
- The CRF was filled out.

### 1.4.2.12. Week 40 (± 5 days)

- The test drug was recovered and dispensed.
- The dosing of the test drug was recorded.
- The concomitant medication was recorded.
- Physical examination.
- The clinical symptoms were recorded.
- The adverse events were recorded.
- The CRF was filled out.

### 1.4.2.13. Week 44 (± 5 days)

- The test drug was recovered and dispensed.
- The dosing of the test drug was recorded.
- The concomitant medication was recorded.
- Physical examination.
- The clinical symptoms were recorded.
- The adverse events were recorded.
- The CRF was filled out.

### 1.4.2.14. Week 48 (± 5 days)

- The test drug was recovered and dispensed.
- The dosing of the test drug was recorded.
- The concomitant medication was recorded.
- Physical examination.
- The clinical symptoms were recorded.
- The adverse events were recorded.
- The CRF was filled out.

### 1.4.2.15. Week 52 (± 5 days)

- The test drug was recovered.
- The dosing of the test drug was recorded.
- The concomitant medication was recorded.
- Physical examination.
- The clinical symptoms were recorded.
- Routine laboratory examination: routine blood test, routine urinalysis, urine pregnancy (women of childbearing age), blood sugar, and the like.
- Serological examination: ALT, AST, GGT, ALP, TP, A, G, TBil, DBil, BUN, Cr, TG, TC, LDL-C, HDL-C, PT, PTA, and AFP.
- Liver transient elastography LSM (kPa).
- Virology examination: HBsAg, anti-HBs, HBeAg, anti-HBe, anti-HBc, and HBV DNA.
- Electrocardiogram.
- Ultrasonic examination: liver, gallbladder, spleen, portal vein width, and/or blood flow rate, and the like.
- Percutaneous liver needle biopsy.
- The adverse events were recorded.
- The CRF was filled out.

### 1.4.3. Additional follow-up

### 1.4.3.1. Additional follow-up conditions

An additional follow-up should be performed in any of the following three cases
- The subject stopped taking the test drug for various reasons, but could follow up on schedule.
- Case dropout: The subject stopped taking the drug and following up.
- After adverse events occurred in the subject, follow-up was increased.

### 1.4.3.2. Additional follow-up contents

- The reason and time for the additional follow-up were recorded.
- The dosing of the test drug was recorded.
- The concomitant medication was recorded.
- The clinical symptoms were recorded, and auxiliary examinations such as physical examination, routine laboratory and biochemical examination, virology examination, electrocardiogram, ultrasound and the like were performed according to actual needs.
- The adverse events were recorded.
- The CRF was filled out.

### 1.5. Observation index

Main observation index: the proportion that the hepatic fibrosis Ishak scores after treatment decreased by not less than 1 point as compared to that before treatment.

Secondary observation indexes:
(1) Negative conversion rate of HBV DNA after treatment (HBV DNA < 20 copies/mL) and decrease level thereof.
(2) The proportion that the liver transient elastography LSM (kPa) value after treatment decreased as compared to that before treatment.
(3) The proportion that the liver tissue inflammation grading after treatment decreased by not less than one grade as compared to that before treatment, and the fibrosis was not worsened.
(4) Improvement of ALT index of liver function.

Safety indexes: vital signs, laboratory indexes, adverse events/adverse effects, and the like.

### 1.6. Statistical protocol and sample size determination

### ● Full analysis set (FAS)

Based on the intention to treat principle, all cases that had been randomized, used the study drug at least once, and had evaluation data after medication, constitute the FAS for this study. The missing data of the part related to the efficacy in FAS were carried forward by a last observation carry forward method (LOCF method). FAS was the main population for efficacy evaluation.

### ● Per protocol set (PPS)

The PPS population was finally determined during blind state inspection of data, and was a secondary population for efficacy evaluation. At least the following criteria were included:
1) The inclusion criteria specified by the test protocol were met.
2) All the planning visits were completed.
3) No drugs or treatments that might affect the efficacy evaluation were used during the trial.
4) The compliance was good (the dosage was between 80%-120%).

### ● Safety evaluation data set (safety set, SS)

All cases that had been randomized, used the study drug at least once, and had safety evaluation data after medication, constitute the safety population for this study.

### ● Processing of missing data and outliers

Missing, unused, or erroneous data, and unreasonable data, were first verified by the central office to supplement and correct the original data table. If the supplement and correction could not be carried out for the data, and the main efficacy index was completely missing, wrong, and contradictory, the data would not be included in the statistical analysis.

The last observation carry forward method (LOCF method) was adopted to fill in partial missing data of the main efficacy index, and the secondary efficacy indexes and the safety indexes were not filled in.

### ● General principles of statistical analysis

All statistical analysis was completed by using SAS9.4 or above version professional statistical analysis software.

For the main efficacy index, the test group and the placebo control group were compared using a superiority test. When P ≤ 0.05, the superiority was considered established. Other tests were performed using a two-sided test. When P ≤ 0.05, the tested difference was considered statistically significant.

The quantitative index was statistically described using the mean, the standard deviation, the median, the minimum value, and the maximum value; the classification index was statistically described using the frequency and relative frequency (percentage).

For the quantitative index, comparisons were made to baseline values during the screening period. Differences inside the groups after treatment were compared using Wilcoxon signed rank test, and differences between the groups after treatment were compared using Kruskal-Wallis H test. The variation of the important efficacy index relative to the baseline in each group was compared using a covariance analysis model. The baseline was taken as a covariate in the model, and the effects of the center and the grouping were considered.

For the enumeration data, the comparisons were made in each group using CMH-χ2 test or Fisher's exact probability method.

### ● Dropout analysis

The number of cases enrolled and completed at each center was summarized, the dropout/exclusion rate and the reasons at each center were separately calculated, and a list of dropped cases was compiled.

The total dropout rate and the dropout rate due to adverse events for each group would be compared using CMH-χ2 test or Fisher's exact probability method.

### ● Demographic data and baseline characteristic analysis

Demographic characteristics (age, height, vital signs, etc.) of the patients were statistically described. The demographic data was compared to other baseline value indexes such as vital signs, disease history, basic treatment, and the like to measure the balance of each group using variance analysis or Kruskal-Wallis H test and CMH-χ2 test or Fisher's exact probability method.

### ● Compliance analysis

The compliance of each group was calculated according to good and poor classes, and then compared and analyzed. Good compliance means 80% ≤ the actual dosage/the specified dosage of a subject ≤ 120%.

### ● Effectiveness analysis

The main time point for efficacy evaluation was at the end of month 12 (for PPS) or at the time of withdrawal from the study (for FAS). In addition, the efficacy at each of the other follow-up time points would also be described.

For the main efficacy index, the test group and the placebo control group were compared using a superiority test. When *P* ≤ 0.05, the superiority was considered established.

The variation of the efficacy index relative to the baseline and the variation of the quantitative index after treatment inside the groups were compared using signed rank test; the variation between the groups was compared using covariance analysis, wherein the baseline was taken as a covariate in the model, the center effect was considered, and the least squares mean (LSMEAN) and 95% CI thereof were calculated; the variation of the classification index was compared using CMH-χ2 test, wherein the center effect was considered, and the 95% CI for the rate difference of two groups was calculated. Evaluation was performed using Logistic regression, if necessary.

### ● Efficacy analysis

### ● Main efficacy index

The difference between the groups of the proportion that the hepatic fibrosis Ishak score after treatment decreased by not less than 1 point as compared to that before treatment was compared using CMH-χ2 test considering the center effect. For the mixed factors which were difficult to control or not controlled before treatment, for example, factors which were unbalanced between the groups before treatment and possibly influenced the efficacy evaluation were used as covariates, the difference of the efficacy between the groups was evaluated using a Logistic regression model, and the center effect was considered.

### ● Secondary efficacy index

1. Negative conversion rate of HBV DNA after treatment between the groups was compared using CMH-χ2 test or Fisher's exact probability method.
2. Liver tissue elasticity reduction after treatment between the groups was compared using Kruskal-Wallis H test.
3. The variation of the liver tissue inflammation grading and scoring after treatment between the groups was compared using Kruskal-Wallis H test; the proportion that the liver tissue inflammation grading after treatment decreased by not less than one grade as compared to that before treatment was compared using CMH-χ2 test or Fisher's exact probability method.
4. The recovery rate of the liver function index after treatment between the groups was compared using CMH-χ2 test or Fisher's exact probability method, and the found values of each index between the groups were compared using variance analysis or Kruskal-Wallis H test.

### ● Safety analysis

Safety indexes include adverse events/adverse effects, laboratory indexes, physical examinations, and the like.
1. The incidence rates of the adverse events/adverse effects of each group were compared using CMH-χ2 test or Fisher's exact probability method, and the relative frequency and severity of all the adverse events in the test, the relationship with the drug, the treatment, the outcome, and the like were described in a table.
2. The physical examinations were described in a table for normal/abnormal changes after the experiment.
3. The laboratory indexes were described in a table for normal/abnormal changes, abnormal exacerbation, and the like before and after the test, and the relationship between the laboratory indexes and the test drug was evaluated. The quantitative indexes were statistically described for each time point measured, the variation inside the groups was compared using Wilcoxon signed rank test, and the variation between the groups was compared using Kruskal-Wallis H test.

### 2. Results:

A total of 168 subjects were enrolled randomly in the test, wherein 21 subjects were dropped/excluded (21/168, 12.50%), 167 subjects were included in the full analysis set (FAS) (43 subjects in the placebo control group, 42 subjects in the 180 mg hydronidone capsule group, 41 subjects in the 270 mg hydronidone capsule group, and 41 subjects in the 360 mg hydronidone capsule group), 147 subjects were included in the per protocol set (PPS) (42 subjects in the placebo control group, 36 subjects in the 180 mg hydronidone capsule group, 35 subjects in the 270 mg hydronidone capsule group, and 34 subjects in the 360 mg hydronidone capsule group), and 168 subjects were included in the safety set (SS) (43 subjects in the placebo control group, 42 subjects in the 180 mg hydronidone capsule group, 42 subjects in the 270 mg hydronidone capsule group, and 41 subjects in the 360 mg hydronidone capsule group).

A total of 8 trial centers were involved in the study. Among the 168 subjects, 121 were male and 47 were female. At baseline, the average age of the subjects was 39.55 ± 10.67 years old. In FAS, the demographic data and general indexes of each group, except for the systolic blood pressure, were not statistically significant (*P* > 0.05) for all differences between the groups, and the balance between the groups was good.

### Main efficacy:

The main efficacy index in this study was the proportion of subjects with hepatic fibrosis Ishak scores that decreased by not less than 1 point after treatment (after 52 weeks of treatment) as compared to that before treatment (baseline).

### 2.1 Diachronic change analysis and change sensitivity analysis of hepatic fibrosis Ishak score

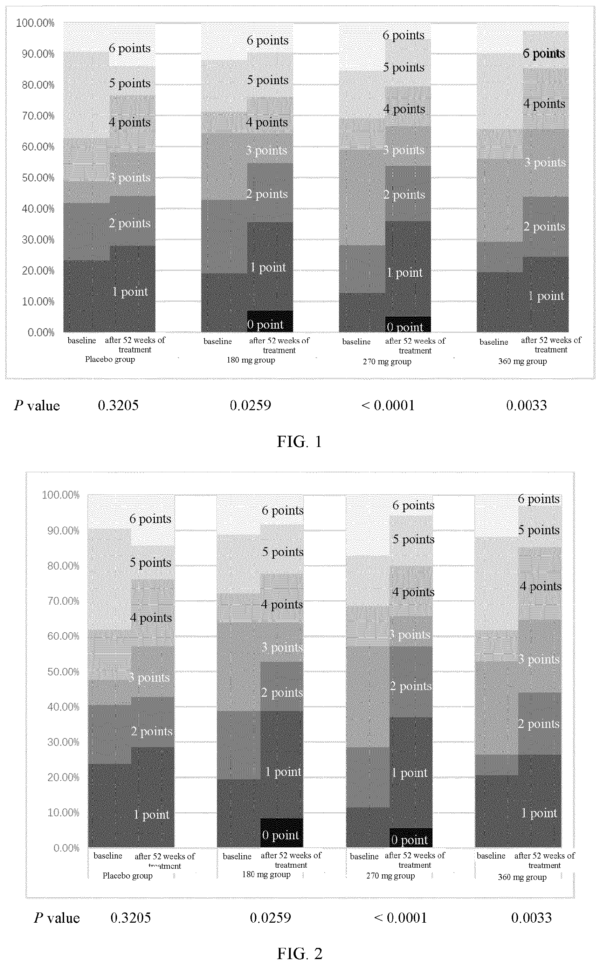

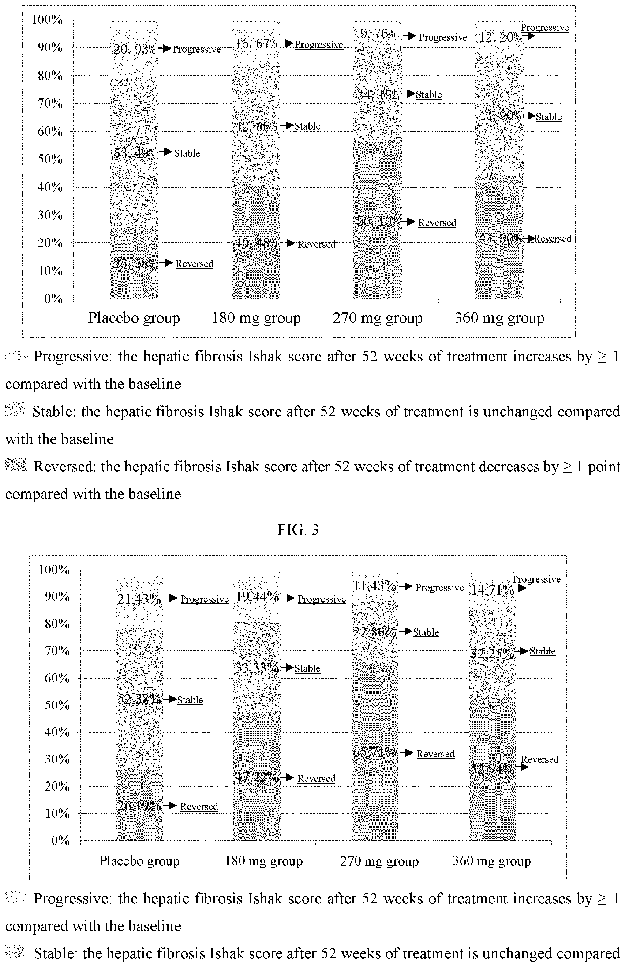

As described above, the pathological evaluation of the center of liver tissue biopsy in this test found that although some subjects were independently judged by the research institution to have liver biopsy hepatic fibrosis with Ishak scores of ≥ 3 at enrollment, which met the inclusion criteria of the test, these subjects actually did not reach the Ishak scores of ≥ 3 according to the pathological evaluation of the center of liver tissue biopsy, including subjects with Ishak scores of ≥ 2 and Ishak scores of ≥ 1. The inventors evaluated all the enrolled subjects, including subjects with Ishak scores of ≥ 1, Ishak scores of ≥ 2, and Ishak scores of ≥ 3. The main efficacy index was the proportion that the hepatic fibrosis Ishak score after treatment (after 52 weeks of treatment) decreased by not less than 1 point as compared to that before treatment (baseline). The FAS and PPS analysis results are shown in Table 1. The FAS analysis results in Table 1 show that differences of baseline hepatic fibrosis Ishak scores between the four groups of subjects were not statistically significant (*P* = 0.8610). After 52 weeks of treatment, the hepatic fibrosis Ishak scores of a certain proportion of the subjects in each group decreased by >_ 1 point compared with the baseline, wherein the variation of the hepatic fibrosis Ishak scores after treatment inside the hydronidone groups was statistically significant (*P* < 0.05), and the difference of the hepatic fibrosis Ishak scores after treatment inside the placebo control group was not statistically significant (*P* = 0.3205), as shown in FIG. 1. The proportion of the subjects with hepatic fibrosis Ishak scores in each group decreased by >_ 1 point compared with the baseline was respectively as follows: 11 (25.58%) in the placebo control group, 17 (40.48%) in the 180 mg hydronidone capsule group, 23 (56.10%) in the 270 mg hydronidone capsule group, and 18 (43.90%) in the 360 mg hydronidone capsule group. Differences between the four groups were statistically significant (*P =* 0.0245). Among the groups, the proportion difference of the subjects with hepatic fibrosis Ishak scores decreased by >_ 1 point between the placebo group and the 270 mg hydronidone group and 95% CI thereof were -30.52% (-48.12%, -9.50%), indicating that 270 mg/day hydronidone capsules could remarkably reverse the hepatic fibrosis of patients compared with the placebo, and the reversing effect of the 270 mg hydronidone capsule group was better than that of 180 mg and 360 mg hydronidone groups, as shown in FIG. 3.

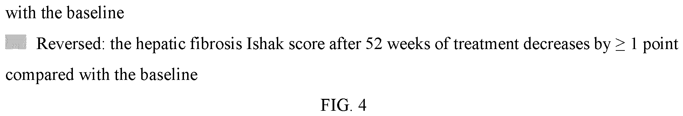

The PPS analysis results in Table 1 show that differences of baseline hepatic fibrosis Ishak scores between the four groups of subjects were not statistically significant (*P* = 0.7361). After 52 weeks of treatment, the hepatic fibrosis Ishak scores of a certain proportion of the subjects in each group decreased by >_ 1 point compared with the baseline, wherein the difference of the hepatic fibrosis Ishak scores after treatment inside the hydronidone groups was statistically significant (P < 0.05), and the variation of the hepatic fibrosis Ishak scores after treatment inside the placebo control group was not statistically significant (*P* = 0.3205), as shown in FIG. 2. The proportion of the subjects with hepatic fibrosis Ishak scores in each group decreased by >_ 1 point compared with the baseline was respectively as follows: 11 (26.19%) in the placebo control group, 17 (47.22%) in the 180 mg hydronidone capsule group, 23 (65.71%) in the 270 mg hydronidone capsule group, and 18 (52.94%) in the 360 mg hydronidone capsule group. Differences between the four groups were statistically significant (P *=* 0.0058). Among the groups, the proportion difference of the subjects with hepatic fibrosis Ishak scores decreased by >_ 1 point among the placebo control group, the 270 mg hydronidone capsule group, and the 360 mg hydronidone capsule group and 95% CI thereof were -39.52% (-56.83%, -17.26%) and - 26.75% (-45.78%, -4.75%), respectively, indicating that 270 mg/day hydronidone capsules and 360 mg/day hydronidone capsules could remarkably reverse the hepatic fibrosis of patients compared with the placebo, and the reversing effect of the 270 mg hydronidone capsule group was the best, as shown in FIG. 4.

**Table 1. Analysis of diachronic changes of hepatic fibrosis Ishak scores**

| **Follow-up time point** | **FAS** | | | | **PPS** | | | |
|---|---|---|---|---|---|---|---|---|
| | **Placebo group Group A (N = 43)** | **360 mg group Group B (N = 41)** | **180 mg group Group C (N = 42)** | **270 mg group Group D (N = 41)** | **Placebo group Group A (N = 42)** | **360 mg group Group B (N = 34)** | **180 mg group Group C (N = 36)** | **270 mg group Group D (N = 35)** |
| Baseline | | | | | | | | |
| N (dropout) | 43(0) | 41(0) | 42(0) | 39(2) | 42(0) | 34(0) | 36(0) | 35(0) |
| Mean (SD) | 3.33(1.78) | 3.39(1.66) | 3.14(1.69) | 3.46(1.62) | 3.36(1.79) | 3.50(1.71) | 3.17(1.66) | 3.51(1.63) |
| Median | 4.00 | 3.00 | 3.00 | 3.00 | 4.00 | 3.00 | 3.00 | 3.00 |
| 1 point, n(%) | 10(23.26) | 8(19.51) | 8(19.05) | 5(12.82) | 10(23.81) | 7(20.59) | 7(19.44) | 4(11.43) |
| 2 points, n(%) | 8(18.60) | 4(9.76) | 10(23.81) | 6(15.38) | 7(16.67) | 2(5.88) | 7(19.44) | 6(17.14) |
| 3 points, n(%) | 3(6.98) | 11(26.83) | 9(21.43) | 12(30.77) | 3(7.14) | 9(26.47) | 9(25.00) | 10(28.57) |
| 4 points, n(%) | 6(13.95) | 4(9.76) | 3(7.14) | 4(10.26) | 6(14.29) | 3(8.82) | 3(8.33) | 4(11.43) |
| 5 points, n(%) | 12(27.91) | 10(24.39) | 7(16.67) | 6(15.38) | 12(28.57) | 9(26.47) | 6(16.67) | 5(14.29) |
| 6 points, n(%) | 4(9.30) | 4(9.76) | 5(11.90) | 6(15.38) | 4(9.52) | 4(11.76) | 4(11.11) | 6(17.14) |
| One-way *CMH-χ*² value | 0.7515 | | | | 1.2708 | | | |
| *P* value | 0.8610 | | | | 0.7361 | | | |
| Follow-up 15 (52 weeks of treatment) | | | | | | | | |
| N (dropout) | 43(0) | 41(0) | 42(0) | 39(2) | 42(0) | 34(0) | 36(0) | 35(0) |
| Mean (SD) | 3.07(1.78) | 2.83(1.45) | 2.71(1.88) | 2.64(1.74) | 3.10(1.79) | 2.82(1.49) | 2.67(1.88) | 2.60(1.77) |
| Median | 3.00 | 3.00 | 2.00 | 2.00 | 3.00 | 3.00 | 2.00 | 2.00 |
| 0 point, n(%) | 0(0.00) | 0(0.00) | 3(7.14) | 2(5.13) | 0(0.00) | 0(0.00) | 3(8.33) | 2(5.71) |
| 1 point, n(%) | 12(27.91) | 10(24.39) | 12(28.57) | 12(30.77) | 12(28.57) | 9(26.47) | 11(30.56) | 11(31.43) |
| 2 points, n(%) | 7(16.28) | 8(19.51) | 8(19.05) | 7(17.95) | 6(14.29) | 6(17.65) | 5(13.89) | 7(20.00) |
| 3 points, n(%) | 6(13.95) | 9(21.95) | 4(9.52) | 5(12.82) | 6(14.29) | 7(20.59) | 4(11.11) | 3(8.57) |
| 4 points, n(%) | 8(18.60) | 8(19.51) | 5(11.90) | 5(12.82) | 8(19.05) | 7(20.59) | 5(13.89) | 5(14.29) |
| 5 points, n(%) | 4(9.30) | 5(12.20) | 6(14.29) | 6(15.38) | 4(9.52) | 4(11.76) | 5(13.89) | 5(14.29) |
| 6 points, n(%) | 6(13.95) | 1(2.44) | 4(9.52) | 2(5.13) | 6(14.29) | 1(2.94) | 3(8.33) | 2(5.71) |
| One-way *CMH-χ*² value | 1.3275 | | | | 2.1633 | | | |
| *P* value | 0.7226 | | | | 0.5392 | | | |
| Baseline-52 weeks of treatment | | | | | | | | |
| N (dropout) | 43(0) | 41(0) | 42(0) | 41(0) | 42(0) | 34(0) | 36(0) | 35(0) |
| Mean (SD) | 0.26(1.60) | 0.56(1.25) | 0.43(1.25) | 0.78(1.11) | 0.26(1.62) | 0.68(1.34) | 0.50(1.34) | 0.91(1.15) |
| Median | 0.00 | 0.00 | 0.00 | 1.00 | 0.00 | 1.00 | 0.00 | 1.00 |
| Signed rank inside groups | 27.00 | 88.50 | 74.50 | 151.00 | 27.00 | 88.50 | 74.50 | 151.00 |
| *P* value | 0.3205 | 0.0033 | 0.0259 | <0.0001 | 0.3205 | 0.0033 | 0.0259 | <0.0001 |
| Ishak score decreased by ≥ 1, n(%) | 11(25.58) | 18(43.90) | 17(40.48) | 23(56.10) | 11(26.19) | 18(52.94) | 17(47.22) | 23(65.71) |
| Ishak score decreased by < 1, n(%) | 32(74.42) | 23(56.10) | 25(59.52) | 18(43.90) | 31(73.81) | 16(47.06) | 19(52.78) | 12(34.29) |
| *CMH-χ*² value | 9.3912 | 12.5339 | | | | | | |
| *P* value | 0.0245 | 0.0058 | | | | | | |
| Rate difference (A-B)% and 95% CI^{#} | -18.32(-36.76, 1.96) | -26.75(-45.78, -4.75) | | | | | | |
| Rate difference (A-C)% and 95% CI | -14.89(-33.32, 4.99) | -21.03(-40.20, 0.26) | | | | | | |
| Rate difference (A-D)% and 95% CI | -30.52(-48.12, -9.50) | -39.52(-56.83, -17.26) | | | | | | |
| Rate difference (B-C)% and 95% CI | 3.43(-17.12, 23.60) | 5.72(-16.89, 27.53) | | | | | | |
| Rate difference (B-D)% and 95% CI | -12.20(-32.01, 9.10) | -12.77(-33.83, 9.98) | | | | | | |
| Rate difference (C-D)% and 95% CI | -15.62(-35.03, 5.65) | -18.49(-38.82, 4.38) | | | | | | |

| | | | | | | | | |
|---|---|---|---|---|---|---|---|---|
| - comparison between the groups is *CMH-χ*² test considering center effect; - rate difference confidence interval is calculated using Newcombe-Wilson method; - missing Ishak central laboratory scoring data of subjects after 52 weeks of treatment observed by the slices in the full analysis set (FAS) are filled in using LOCF method; - missing baseline Ishak central laboratory scoring data of subjects observed by the slices will be treated as invalid; - "baseline-52 weeks of treatment" refers to the change in the two scores obtained by subtracting the Ishak score after 52 weeks of treatment from the Ishak score at baseline. | | | | | | | | |

**Table 2. Sensitivity analysis of diachronic changes of hepatic fibrosis Ishak scores after 52 weeks of treatment**

| | **FAS** | | | | **PPS** | | | |
|---|---|---|---|---|---|---|---|---|
| **Follow-up time point** | **Placebo group Group A (N = 43)** | **360 mg group Group B (N = 41)** | **180 mg group Group C (N** = **42)** | **270 mg group Group D (N = 41)** | **Placebo group Group A (N = 42)** | **360 mg group Group B (N = 34)** | **180 mg group Group C (N = 36)** | **270 mg group Group D (N = 35)** |
| N (dropout) | 43(0) | 41(0) | 42(0) | 41(0) | 42(0) | 34(0) | 36(0) | 35(0) |
| Progressive Ishak score | 9(20.93) | 5(12.20) | 7(16.67) | 4(9.76) | 9(21.43) | 5(14.71) | 7(19.44) | 4(11.43) |
| Stable Ishak score | 23(53.49) | 18(43.90) | 18(42.86) | 14(34.15) | 22(52.38) | 11(32.35) | 12(33.33) | 8(22.86) |
| Reversed Ishak score | 11(25.58) | 18(43.90) | 17(40.48) | 23(56.10) | 11(26.19) | 18(52.94) | 17(47.22) | 23(65.71) |
| *CMH-χ*² value^{[1]} | 3.1890 | | | | 7.7644 | | | |
| *P* value^{[1]} | 0.3634 | | | | 0.0511 | | | |
| *CMH-χ*² value^{[2]} | 2.4042 | | | | 1.6214 | | | |
| *P* value^{[2]} | 0.4929 | | | | 0.6545 | | | |

| | | | | | | | | |
|---|---|---|---|---|---|---|---|---|
| - progressive Ishak score: the hepatic fibrosis Ishak score after 52 weeks of treatment increases by >_ 1 compared with the baseline; stable Ishak score: the hepatic fibrosis Ishak score after 52 weeks of treatment is unchanged compared with the baseline; reversed Ishak score: the hepatic fibrosis Ishak score after 52 weeks of treatment decreases by >_ 1 point compared with the baseline; - missing Ishak central laboratory scoring data of subjects after 52 weeks of treatment observed by the slices in the full analysis set (FAS) are filled in using LOCF method; - missing baseline Ishak central laboratory scoring data of subjects observed by the slices will be treated as invalid, i.e., subjects are considered stable in Ishak score, and the hepatic fibrosis Ishak score after 52 weeks of treatment is considered unchanged compared with the baseline. - ^{[1]} represents the comparative difference of the distribution of subjects with stable Ishak score between the groups; ^{[2]} represents the comparative difference of the distribution of subjects with progressive Ishak score between the groups. | | | | | | | | |

Statistical results of progressive hepatic fibrosis Ishak scores after 52 weeks of treatment compared with the baseline (the Ishak scores increase by ≥ 1) are shown in Table 2 and FIGs. 3 and 4. The results show that: the placebo control group had more subjects with worsened hepatic fibrosis Ishak scores than the other three groups, and the difference between the groups in FAS was not statistically significant (*P* = 0.4929). The proportion of the subjects with worsened hepatic fibrosis Ishak scores in each group compared with the baseline was respectively as follows: 9 (20.93%) in the placebo group, 7 (16.67%) in the 180 mg hydronidone capsule group, 4 (9.76%) in the 270 mg hydronidone capsule group, and 5 (12.20%) in the 360 mg hydronidone capsule group. The results of PPS were similar to those of FAS, and the difference between the groups thereof was not statistically significant (P = 0.6545). The proportion of the subjects with progressive hepatic fibrosis Ishak scores in each group compared with the baseline was respectively as follows: 9 (21.43%) in the placebo control group, 7 (19.44%) in the 180 mg hydronidone capsule group, 4 (11.43%) in the 270 mg hydronidone capsule group, and 5 (14.71%) in the 360 mg hydronidone capsule group.

Statistical results of unchanged hepatic fibrosis Ishak scores after 52 weeks of treatment compared with the baseline are shown in Table 2 and FIGs. 1 and 2. The results show that: the placebo control group had more subjects with stable hepatic fibrosis Ishak scores than the other three groups, and the difference between the groups in FAS was not statistically significant (*P* = 0.3634). The proportion of the subjects with unchanged hepatic fibrosis Ishak scores in each group compared with the baseline was respectively as follows: 23 (53.49%) in the placebo control group, 18 (42.86%) in the 180 mg hydronidone capsule group, 14 (34.15%) in the 270 mg hydronidone capsule group, and 18 (43.90%) in the 360 mg hydronidone capsule group. The difference between the groups in PPS was not statistically significant (*P* = 0.0511). The proportion of the subjects with unchanged hepatic fibrosis Ishak scores in each group compared with the baseline was respectively as follows: 22 (52.38%) in the placebo control group, 12 (33.33%) in the 180 mg hydronidone capsule group, 8 (22.86%) in the 270 mg hydronidone capsule group, and 11 (32.35%) in the 360 mg hydronidone capsule group.

In conclusion, after 52 weeks of treatment, the hydronidone group could reverse the hepatic fibrosis of subjects compared with the placebo group, wherein the 270 mg/day hydronidone capsules had the best effect on reversing the hepatic fibrosis of patients.

### 2.2 Statistical analysis results of patients with Ishak scores ≥ 2

The inventors also evaluated the effect of hydronidone in hepatic fibrosis patients with baseline Ishak scores of ≥ 2. The data are shown in Table 3.

**Table 3. Analysis of results of *baseline ISHAK scores ≥ 2, group B/C/D combination compared with group A, and ISHAK scores decreased by ≥ 1***

| **Follow-up time point** | **FAS** | | **PPS** | |
|---|---|---|---|---|
| | **Group A** | **Group** B/C/D | **Group A** | **Group** B/C/D |
| Screening period | | | | |
| N (dropout) | 33(0) | 101(0) | 32(0) | 87(0) |
| 2 points, n(%) | 8(24.24) | 20(19.80) | 7(21.88) | 15(17.24) |
| 3 points, n(%) | 3(9.09) | 32(31.68) | 3(9.38) | 28(32.18) |
| 4 points, n(%) | 6(18.18) | 11(10.89) | 6(18.75) | 10(11.49) |
| 5 points, n(%) | 12(36.36) | 23(22.77) | 12(37.50) | 20(22.99) |
| 6 points, n(%) | 4(12.12) | 15(14.85) | 4(12.50) | 14(16.09) |
| One-way *CMH-χ*² value | 0.6175 | | 0.5402 | |
| *P* value | 0.4320 | | 0.4624 | |
| Follow-up 15 (52 weeks of treatment) | | | | |
| N (dropout) | 33(0) | 101(0) | 32(0) | 87(0) |
| 0 point, n(%) | 0(0.00) | 5(4.95) | 0(0.00) | 5(5.75) |
| 1 point, n(%) | 5(15.15) | 20(19.80) | 5(15.63) | 20(22.99) |
| 2 points, n(%) | 6(18.18) | 17(16.83) | 5(15.63) | 12(13.79) |
| 3 points, n(%) | 5(15.15) | 18(17.82) | 5(15.63) | 14(16.09) |
| 4 points, n(%) | 7(21.21) | 17(16.83) | 7(21.88) | 16(18.39) |
| 5 points, n(%) | 4(12.12) | 17(16.83) | 4(12.50) | 14(16.09) |
| 6 points, n(%) | 6(18.18) | 7(6.93) | 6(18.75) | 6(6.90) |
| One-way *CAM-χ*² value | 2.2462 | | 2.9184 | |
| *P* value | 0.1339 | | 0.0876 | |
| Screening period-follow-up 15 | | | | |
| N (dropout) | 33(0) | 101(0) | 32(0) | 87(0) |
| Ishak score decreased by ≥ 1, n(%) | 11(33.33) | 58(57.43) | 11(34.38) | 58(66.67) |
| Ishak score decreased by < 1, n(%) | 22(66.67) | 43(42.57) | 21(65.63) | 29(33.33) |
| *CMH-χ*² value | 5.7370 | | 9.9292 | |
| *P* value | 0.0166 | | 0.0016 | |
| Rate difference (A-B)% and 95% CI^{#} | -24.09(-40.50, -4.45) | | -32.29(-48.91, -12.08) | |

| | | | | |
|---|---|---|---|---|
| Note: "screening period-follow-up 15" refers to the change in the two scores obtained by subtracting the Ishak score of follow-up 15 from the Ishak score of the screening period. | | | | |

### 2.3 Main efficacy evaluation of patients with significant fibrosis

### Statistical analysis results of patients with Ishak scores ≥ 3

The pathological evaluation of the center of liver tissue biopsy in this test found that although some subjects were independently judged by the research institution to have liver biopsy hepatic fibrosis with Ishak scores of ≥ 3 at enrollment, which met the inclusion criteria of the test, these subjects actually did not reach the Ishak scores of ≥ 3 according to the pathological evaluation of the center of liver tissue biopsy, including subjects with Ishak scores of ≥ 2 and Ishak scores of ≥ 1. After being reviewed and confirmed by blind meetings, the pathological score of the center was only used for judging the main efficacy. Therefore, the test result was more in line with the real situation, and the therapeutic effect of the test drug on the hepatic fibrosis in each stage could be observed.

According to the pathological Ishak score of the liver tissue in the test, the significant reversal efficacy of the 270 mg hydronidone group on hepatic fibrosis was observed, and meanwhile, the liver transient elastography examination also reflected the optimal trend of the reversal of the stiffness of the liver by the 270 mg hydronidone group, which was consistent with the pathological score. Each hydronidone test group had no influence on the reduction of the virus titer by entecavir and the ALT recovery. The inventors supplementarily analyzed the main efficacy evaluation of patients with pathological Ishak scores of the center of liver tissue biopsy of ≥ 3 (namely significant fibrosis), as shown in Table 4. The results were consistent with the above analysis results, and the 270 mg hydronidone group had the most significant effect on reversing hepatic fibrosis.

**Table 4. Analysis of results for subjects with baseline Ishak scores of ≥ 3 decreased by >_ 1 after 52 weeks of treatment**

| **Follow-up time point** | **FAS** | | | | **PPS** | | | |
|---|---|---|---|---|---|---|---|---|
| | **Placebo group Group A** | **360 mg group Group B** | **180 mg group Group C** | **270 mg group Group D** | **Placebo group Group A** | **360 mg group Group B** | **180 mg group Group C** | **270 mg group Group D** |
| Screening period | | | | | | | | |
| N (dropout) | 25(0) | 29(0) | 24(0) | 28(0) | 25(0) | 25(0) | 22(0) | 25(0) |
| 3 points, n(%) | 3(12.00) | 11(37.93) | 9(37.50) | 12(42.86) | 3(12.00) | 9(36.00) | 9(40.91) | 10(40.00) |
| 4 points, n(%) | 6(24.00) | 4(13.79) | 3(12.50) | 4(14.29) | 6(24.00) | 3(12.00) | 3(13.64) | 4(16.00) |
| 5 points, n(%) | 12(48.00) | 10(34.48) | 7(29.17) | 6(21.43) | 12(48.00) | 9(36.00) | 6(27.27) | 5(20.00) |
| 6 points, n(%) | 4(16.00) | 4(13.79) | 5(20.83) | 6(21.43) | 4(16.00) | 4(16.00) | 4(18.18) | 6(24.00) |
| One-way CMH-*χ*² value | 2.8391 | | | | 2.4642 | | | |
| *P* value | 0.4171 | | | | 0.4818 | | | |
| Follow-up 15 (52 weeks of treatment) | | | | | | | | |
| N (dropout) | 25(0) | 29(0) | 24(0) | 28(0) | 25(0) | 25(0) | 22(0) | 25(0) |
| 0 point, n(%) | 0(0.00) | 0(0.00) | 1(4.17) | 2(7.14) | 0(0.00) | 0(0.00) | 1(4.55) | 2(8.00) |
| 1 point, n(%) | 3(12.00) | 4(13.79) | 4(16.67) | 4(14.29) | 3(12.00) | 4(16.00) | 4(18.18) | 4(16.00) |
| 2 points, n(%) | 2(8.00) | 4(13.79) | 1(4.17) | 4(14.29) | 2(8.00) | 4(16.00) | 1(4.55) | 4(16.00) |
| 3 points, n(%) | 5(20.00) | 8(27.59) | 3(12.50) | 5(17.86) | 5(20.00) | 6(24.00) | 3(13.64) | 3(12.00) |
| 4 points, n(%) | 5(20.00) | 7(24.14) | 5(20.83) | 5(17.86) | 5(20.00) | 6(24.00) | 5(22.73) | 5(20.00) |
| 5 points, n(%) | 4(16.00) | 5(17.24) | 6(25.00) | 6(21.43) | 4(16.00) | 4(16.00) | 5(22.73) | 5(20.00) |
| 6 points, n(%) | 6(24.00) | 1(3.45) | 4(16.67) | 2(7.14) | 6(24.00) | 1(4.00) | 3(13.64) | 2(8.00) |
| One-way *CMH-χ*² value | 3.4946 | | | | 3.4306 | | | |
| *P* value Screening period-follow-up 15 | 0.3215 | | | | 0.3299 | | | |
| N (dropout) | 25(0) | 29(0) | 24(0) | 28(0) | 25(0) | 25(0) | 22(0) | 25(0) |
| Ishak score decreased by ≥ 1, n(%) | 9(36.00) | 17(58.62) | 13(54.17) | 18(64.29) | 9(36.00) | 17(68.00) | 13(59.09) | 18(72.00) |
| Ishak score decreased by < 1, n(%) | 16(64.00) | 12(41.38) | 11(45.83) | 10(35.71) | 16(64.00) | 8(32.00) | 9(40.91) | 7(28.00) |
| *CMH-χ*² value | 4.6531 | | | | 7.9518 | | | |
| *P* value | 0.1990 | | | | 0.0470 | | | |
| Rate difference (A-B)% and 95% CI^{#} | -22.62(-44.98, 3.82) | | | | -32.00(-53.61, -4.37) | | | |
| Rate difference (A-C)% and 95% CI | -18.17(-42.04, 9.11) | | | | -23.09(-46.75, 5.09) | | | |
| Rate difference (A-D)% and 95% CI | -28.29(-50.04, -1.45) | | | | -36.00(-56.89, -8.38) | | | |
| Rate difference (B-C)% and 95% CI | 4.45(-20.88, 29.28) | | | | 8.91(-17.46, 34.07) | | | |
| Rate difference (B-D)% and 95% CI | -5.67(-29.01, 18.67) | | | | -4.00(-27.91, 20.54) | | | |
| Rate difference (C-D)% and 95% CI | -10.12(-34.40, 15.62) | | | | -12.91(-37.46, 13.45) | | | |

| | | | | | | | | |
|---|---|---|---|---|---|---|---|---|
| Note: "screening period-follow-up 15" refers to the change in the two scores obtained by subtracting the Ishak score of follow-up 15 from the Ishak score of the screening period. | | | | | | | | |

The inventors compared the effect of the high-dose groups (270 mg group + 360 mg group) in combination of hydronidone with that of the placebo group in hepatic fibrosis patients with baseline Ishak scores of ≥ 3 (i.e. significant fibrosis). The results show that fibrosis was significantly reversed with high and medium doses of hydronidone (*P* < 0.05) in hepatic fibrosis patients with Ishak scores of ≥ 3, 4, or = 6. The results suggest that hydronidone had efficacy on significant fibrosis, progressive fibrosis, and cirrhosis. The effect data are shown in Table 5.

**Table 5. Analysis of results for subjects with baseline Ishak scores of ≥ 3 decreased by >_ 1 in a combination of high and medium dose groups after 52 weeks of treatment**

| | **Baseline-after 52 weeks of treatment** | **FAS** | | **PPS** | |
|---|---|---|---|---|---|
| | | **Placebo group** | **270 mg group + 360 mg group** | **Placebo group** | **270 mg group + 360 mg group** |
| Baseline Ishak score ≥ 3 Significant fibrosis | N (dropout) | 25(0) | 57(0) | 25(0) | 50(0) |
| | Ishak score decreased by ≥ 1, n(%) | 9(36.00) | 35(61.40) | 9(36.00) | 35(70.00) |
| | *P* value | 0.0348 | | 0.0051 | |
| | Rate difference (A-B)% and 95% CI | -25.40(-44.93,-2.00) | | -34.00(-53.15,-10.15) | |
| Baseline Ishak score ≥ 4 Progressive fibrosis | N (dropout) | 22(0) | 34(0) | 22(0) | 31(0) |
| | Ishak score decreased by ≥1, n(%) | 9(40.91) | 22(64.71) | 9(40.91) | 22(70.97) |
| | *P* value | 0.083 | | 0.0302 | |
| | Rate difference (A-B)% and 95% CI | -23.80(-46.21,2.59) | | -30.06(-51.94,-3.18) | |
| Baseline Ishak score ≥ 5 Early cirrhosis | N (dropout) | 16(0) | 26(0) | 16(0) | 24(0) |
| | Ishak score decreased by ≥ 1, n(%) | 8(50.00) | 17(65.38) | 8(50.00) | 17(70.83) |
| | *P* value | 0.3298 | | 0.188 | |
| | Rate difference (A-B)% and 95% CI | -15.38(-42.13,13.79) | | -20.83(-47.05,8.90) | |
| Baseline Ishak score = 6 Cirrhosis | N (dropout) | 4(0) | 10(0) | 4(0) | 10(0) |
| | Ishak score decreased by ≥ 1, n(%) | 1(25.00) | 9(90.00) | 1(25.00) | 9(90.00) |
| | Fisher's exact probability *P* value | 0.0410 | | 0.0410 | |
| | Rate difference (A-B)% and 95% CI | -65.00(-87.03,-10.74) | | -65.00(-87.03,-10.74) | |

| | | | | | |
|---|---|---|---|---|---|
| Note: "baseline-after 52 weeks of treatment" refers to the change in the two scores obtained by subtracting the Ishak score after 52 weeks of treatment from the Ishak score at baseline. | | | | | |

### Secondary efficacy:

### Decline rate of liver transient elastography LSM (kPa)

Differences of baseline liver transient elastography LSM (kPa) values between the four groups were not statistically significant (*P* = 0.6906). The liver transient elastography LSM (kPa) values of each group after 52 weeks of treatment were reduced, and the variation of the liver transient elastography LSM (kPa) assay values after treatment inside the groups was statistically significant (*P* < 0.01). The mean decline rate of the liver transient elastography LSM (kPa) of the hydronidone group after 52 weeks of treatment was higher than that of the control group, and the mean decline rate of the 270 mg hydronidone capsule group was the largest, which was consistent with the pathological scoring results, but the difference between the groups in FAS was not statistically significant (*P* = 0.7311). The mean decline rate (standard deviation) of the liver transient elastography LSM (kPa) of each group was respectively as follows: 10.16% (63.19) in the placebo control group, 18.30% (29.65) in the 180 mg hydronidone capsule group, 24.62% (31.23) in the 270 mg hydronidone capsule group, and 18.98% (33.96) in the 360 mg hydronidone capsule group. The analysis results of PPS were similar to those of FAS.

### Amelioration of liver tissue inflammation

Differences of baseline liver tissue inflammation grading and scoring between the four groups were not statistically significant (FAS: *P* = 0.9123, and PPS: *P* = 0.7984). The liver tissue inflammation of each group after 52 weeks of treatment was ameliorated, and the variation of the liver tissue inflammation scoring after treatment inside the groups was statistically significant (*P* < 0.001). The proportion of the subjects with the liver tissue inflammation ameliorated in each group was respectively as follows: 22 (52.38%) in the placebo control group, 21 (58.33%) in the 180 mg hydronidone capsule group, 20 (57.14%) in the 270 mg hydronidone capsule group, and 19 (55.88%) in the 360 mg hydronidone capsule group. The liver tissue inflammation of hydronidone groups was ameliorated, but the differences between the four groups were not statistically significant (*P* = 0.9570). The results of PPS were consistent with those of FAS after 52 weeks of treatment.

### Negative conversion rate of HBV DNA

Differences of baseline HBV DNA positive rates between the four groups were not statistically significant (*P* = 0.1474). The proportion of the subjects with HBV DNA substantially converted to negative after 52 weeks of treatment in each group was respectively as follows: 40 (95.24%) in the placebo control group, 34 (94.44%) in the 180 mg hydronidone capsule group, 34 (97.14%) in the 270 mg hydronidone capsule group, and 32 (94.12%) in the 360 mg hydronidone capsule group. The differences of negative conversion rates between the four groups were not statistically significant (*P =* 0.9569). The analysis results of PPS were similar to those of FAS.

### ALT recovery rate

The FAS analysis results show that differences of baseline ALT assay values between the four groups were not statistically significant (*P* = 0.3058). The ALT assay values of each group after 52 weeks of treatment were reduced, and the variation of ALT after treatment inside the groups was statistically significant (*P* < 0.001). The mean (standard deviation) of variation of the ALT index after 52 weeks of treatment in each group compared with the baseline was respectively as follows: 64.15 (73.63) in the placebo control group, 44.71 (53.50) in the 180 mg hydronidone capsule group, 66.47 (78.13) in the 270 mg hydronidone capsule group, and 43.58 (59.87) in the 360 mg hydronidone capsule group. But the differences of the variation between the groups were not statistically significant (*P* = 0.5132). The analysis results of PPS were similar to those of FAS.

The FAS analysis results show that after 52 weeks of treatment, the proportion of the subjects with a certain clinical significance of the ALT indexes determined to turn normal from abnormal (baseline) in each group was respectively as follows: 21 (87.50%) in the placebo control group, 15 (78.95%) in the 180 mg hydronidone capsule group, 18 (85.71%) in the 270 mg hydronidone capsule group, and 16 (88.89%) in the 360 mg hydronidone capsule group. The analysis results of PPS were similar to those of FAS.

### Safety:

The safety of hydronidone was good. In the test process, differences of adverse events, adverse effects, and SAE incidence rates between the four groups were not statistically different.

The above examples illustrate the embodiments of the present disclosure. However, the present disclosure is not limited to the embodiments described above. Any modification, equivalent, improvement, and the like made without departing from the spirit and principle of the present disclosure shall fall within the protection scope of the present disclosure.

## Claims

1. Use of a compound of formula (I), a solvate, a hydrate, a prodrug, or a pharmaceutically acceptable salt thereof for manufacturing a medicament for the treatment and/or prevention of chronic hepatitis B with hepatic fibrosis,

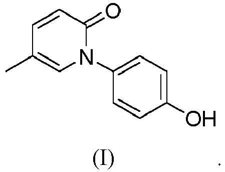

2. The use as claimed in claim 1, wherein the chronic hepatitis B with hepatic fibrosis is portal area fibrosis, significant hepatic fibrosis, progressive hepatic fibrosis, or cirrhosis;
preferably, the cirrhosis is compensated cirrhosis or decompensated cirrhosis.

3. The use as claimed in claim 2, wherein the portal area fibrosis can be **characterized by** one, two, or more of the following:
(1) the portal area fibrosis is characterized via an Ishak scoring system, which means hepatic fibrosis with an Ishak score of ≥ 1, or hepatic fibrosis with an Ishak score of about 1-3;
(2) the portal area fibrosis is characterized via a Scheuer scoring system, which can mean, for example, hepatic fibrosis with a Scheuer score of about ≥ 1, or hepatic fibrosis with a Scheuer score of about 1-2;
(3) the portal area fibrosis is characterized via a METAVIR scoring system, which means hepatic fibrosis with a METAVIR score of about ≥ 1, or hepatic fibrosis with a METAVIR score of about 1-2; and/or
(4) the portal area fibrosis is characterized via a Knodell scoring system, which means hepatic fibrosis with a Knodell score of about ≥ 1, or hepatic fibrosis with a Knodell score of about 1-2.

4. The use as claimed in any one of claims 1-3, wherein the significant hepatic fibrosis can be **characterized by** one, two, or more of the following:
(1) the significant hepatic fibrosis is characterized via an Ishak scoring system, which means hepatic fibrosis with an Ishak score of ≥ 3 or ≥ 4; for example, the Ishak score is about 3-6;
(2) the significant hepatic fibrosis is characterized via a Scheuer scoring system, which means hepatic fibrosis with a Scheuer score of about ≥ 2 or ≥ 3; for example, the Scheuer score is about 2-4;
(3) the significant hepatic fibrosis is characterized via a METAVIR scoring system, which means hepatic fibrosis with a METAVIR score of about ≥ 2 or ≥ 3; for example, the METAVIR score is about 2-4;
(4) the significant hepatic fibrosis is characterized via a Knodell scoring system, which means hepatic fibrosis with a Knodell score of about ≥ 2 or ≥ 3; for example, the Knodell score is about 2-4; and/or
(5) the significant hepatic fibrosis is **characterized by** a liver stiffness measurement, which means hepatic fibrosis with an LSM of about ≥ 5.8 kPa, for example, an LSM of about 5.8-12.4 kPa.

5. The use as claimed in any one of claims 1-4, wherein the progressive hepatic fibrosis can be **characterized by** one, two, or more of the following:
(1) the progressive hepatic fibrosis is characterized via an Ishak scoring system, which means, for example, hepatic fibrosis with an Ishak score of ≥ 4 or ≥ 5; for example, the Ishak score is about 4-6;
(2) the progressive hepatic fibrosis is characterized via a Scheuer scoring system, which means hepatic fibrosis with a Scheuer score of about ≥ 3, or about 3-4;
(3) the progressive hepatic fibrosis is characterized via a METAVIR scoring system, which means hepatic fibrosis with a METAVIR score of about ≥ 3, or about 3-4;
(4) the progressive hepatic fibrosis is characterized via a Knodell scoring system, which means hepatic fibrosis with a Knodell score of about ≥ 2 or ≥ 3, or hepatic fibrosis with a Knodell score of about 2-4 or 2-3; and/or
(5) the progressive hepatic fibrosis is **characterized by** a liver stiffness measurement, which means hepatic fibrosis with an LSM of about ≥ 9.0 kPa or ≥ 10.0 kPa under the condition that bilirubin is normal and ALT is < 5 ULN, or hepatic fibrosis with an LSM of about ≥ 6.0 kPa under the condition that bilirubin is normal and ALT is normal.

6. The use as claimed in any one of claims 1-5, wherein the cirrhosis can be **characterized by** one, two, or more of the following:
(1) the cirrhosis is characterized via an Ishak scoring system, which means cirrhosis with an Ishak score of ≥ 5, or about 5-6;
(2) the cirrhosis is characterized via a Scheuer scoring system, which means cirrhosis with a Scheuer score of about >_ 4;
(3) the cirrhosis is characterized via a METAVIR scoring system, which means cirrhosis with a METAVIR score of about ≥ 4;
(4) the cirrhosis is characterized via a Knodell scoring system, which means cirrhosis with a Knodell score of about ≥ 4; and/or
(5) the cirrhosis is **characterized by** a liver stiffness measurement, which means cirrhosis with an LSM of about ≥ 12.0 kPa or about ≥ 13.0 kPa.

7. The use as claimed in any one of claims 1-6, wherein the chronic hepatitis B with hepatic fibrosis has not progressed to liver cancer.

8. The use as claimed in any one of claims 1-7, wherein the compound of formula (I) is used in an amount of 50-500 mg/day, such as 80-450 mg/day, 100-400 mg/day, or 120-360 mg/day; preferably, the medicament is administered 1-5 times daily, for example, 3 times daily.

9. The use as claimed in any one of claims 1-8, wherein the pharmaceutically acceptable salt includes salts formed by an ester formed by the compound of formula (I) with an organic acid selected from propionic acid, oxalic acid, malonic acid, succinic acid, fumaric acid, maleic acid, lactic acid, malic acid, tartaric acid, and citric acid, or an acidic amino acid selected from aspartic acid and glutamic acid, with an inorganic base, including sodium, potassium, calcium, aluminium and ammonium salts, or salts formed with an organic base, including methylamine, ethylamine and ethanolamine salts; or salts formed by an ester formed by the compound of formula (I) with a basic amino acid selected from lysine, arginine, and ornithine, with an inorganic acid selected from hydrochloric acid, hydrobromic acid, hydrofluoric acid, sulfuric acid, nitric acid, and phosphoric acid, or salts formed with an organic acid selected from formic acid, acetic acid, picric acid, methanesulfonic acid, and ethanesulfonic acid.

10. The use as claimed in any one of claims 1-9, wherein the medicament further comprises one or more pharmaceutically acceptable carriers or excipients.

11. A method of treatment and/or prevention of chronic hepatitis B with hepatic fibrosis, comprising the step of administering to a patient in need thereof a therapeutically or prophylactically effective amount of the compound of formula (I), the solvate, the hydrate, the prodrug, or the pharmaceutically acceptable salt thereof as claimed in claim 1.

12. A pharmaceutical composition, comprising the compound of formula (I) or the pharmaceutically acceptable salt thereof as claimed in claim 1, lactose monohydrate, silica, and magnesium stearate.

13. The pharmaceutical composition as claimed in claim 12, wherein, in weight percentage, the compound of formula (I) or the pharmaceutically acceptable salt thereof has a content of 20-30%, the lactose monohydrate has a content of 70-80%, the silica has a content of 0.1-1%, and the magnesium stearate has a content of 0.1-1%.
